# EUROPEAN PATENT APPLICATION

(11) **EP 3 726 536 A1**
(43) Date of publication of application: **21.10.2020**
(21) Application number: 20170132.3
(22) Date of filing: 17.04.2020
(51) Int. Cl.: G16H 50/30

(54) **CLASSIFICATION METHOD OF GLAUCOMA RELATED DAMAGES**

(30) Priority: 19.04.2019 IT 201900006120
(71) Applicant: Artema SRL, 31100 Treviso (IT)
(72) Inventor: BRUSINI, Paolo, 33100 UDINE (IT)
(74) Representative: Modiano, Micaela Nadia

(57) **Abstract**

A method for classifying the damage due to the glaucoma of a patient on the basis of an existing data set which comprises:
- a value MD of average loss of sensitivity of a visual field of the patient with respect to a subject without glaucoma of similar age,
- a value PSD of standard deviation of the MD of the patient,
- a value RNFL of average thickness of the layer of fibers of the optic nerve (Retinal Nerve Fiber Layer) in the inferior quadrant of said patient, and
- a value RNFL of average thickness of the layer of fibers of the optic nerve in the superior quadrant of said patient,
the method comprising the following steps:
1) determining a score that indicates the severity of the damage to the visual field of said patient on the basis of said MD and PSD values, in stages of rising severity of the damage to the visual field,
2) determining a score that indicates the severity of the damage to the optic nerve of the patient on the basis of said values of average thickness of the layer of fibers of the optic nerve in the inferior and superior quadrants of the patient,
3) determining a score of the global damage due to glaucoma of the patient on the basis of the score that indicates the severity of the damage to the visual field and of the score that indicates the severity of the damage to the optic nerve of the patient, which have been determined in steps 1) and 2).

## Description

The present invention relates to a method for classifying global damage due to glaucoma.

Various methods for classifying glaucomatous damage have been studied and proposed and mostly use data obtained with the so-called "Computerized Automated Perimetry" ("CAP") method (Katz J, Tielsch JM, Quigley HA, Sommer A: Automated perimetry detects visual field loss before manual Goldmann perimetry. Ophthalmology. 1995 Jan; 102(1):21-6. Mutlukan E: Diffuse and localised visual field defects to automated perimetry in primary open angle glaucoma. Eye (Lond). 1995;9 (Pt 6):745-50).

In the CAP method the patient is subjected to a perimetry test that allows to define the perceptive level inside the visual field (differential light sensitivity) by using light stimuli evenly distributed in a substantially circular area.

The greater or lower sensitivity in the perception of the light stimulus allows to formulate a diagnosis regarding the presence, if any, of any glaucomatous damage and its severity. Perimetry indices are extrapolated mathematically from the numerical data obtained by means of the perimetry test and allow to formulate an approximate evaluation of the severity and distribution of the damage.

The most important perimetry indices that can be obtained by means of CAP are the "MD" ("Mean Defect" or "Mean Deviation") index, which provides information on the global or generalized level of loss of visual sensitivity, and the "LV", "Loss Variance" (or "CLV", "Corrected Loss Variance") index, or its square root "PSD", "Pattern Standard Deviation" (or "CPSD", "Corrected Pattern Standard Deviation"), which provide information on the inhomogeneity of the defects in the visual field.

Italian Patent Application No. UD95A000007 filed on 23 January 1995 describes a standardized system (and a corresponding device) for classification and staging of defects of the visual field, suitable for classifying glaucomatous damage starting from at least two perimetry indices obtained by a test according to CAP.

The first perimetry index, particularly MD index, provides an indication of the global or generalized damage, while the second perimetry index, particularly CPSD/PSD (or CLV/LV) index, provides an indication of the homogeneity of spatial distribution of the damage in the visual field.

The classification system provides for the preparation of a chart with at least two coordinates:
- the values of the MD index are identified on a first coordinate,
- the values of the CPSD/PSD (or CLV/LV) index are identified on the second coordinate.

The chart is partitioned by a grid which is adapted to define a plurality of contiguous classes in relation to the severity and type of the found glaucomatous damage.

The positioning of the specific glaucomatous damage, as defined by the intersection between the values of the two perimetry indices shown in the chart within a specific class, allows a precise and straightforward definition of the severity level and of the degree of more or less accentuated localization of said damage.

"Clinical use of a new method for visual field damage classification in glaucoma", P. Brusini, European Journal of Ophthalmology, Vol. 6, 1996, p.402-407, describes a method, known as "Glaucoma Staging System" (GSS), for classifying visual field damage in glaucoma, created by examining several hundred computerized glaucomatous visual fields obtained with the Humphrey 30-2 (Anderson DR: Automated static perimetry, Mosby Year Book, Inc, 1992, p. 162-169) and Octopus G1 (or G1X) (Flammer AJ, Jenni A, Bebie H, Keller B: The Octopus glaucoma G1 program, Glaucoma, 1987; 9:67-72) threshold tests.

Statistical data of Humphrey Statpac 2 (in particular, the total deviation and standard deviation probability maps of the model, global indices and the Glaucoma Hemifield test, GHT), Bebie curve (Bebie H, Flammer J, Bebie T: The cumulative defect curve: separation of local and diffuse components of visual field damage. Graefes Arch Clin Exp Ophthalmol, 1989; 227:9-12) and the Octopus 1-2-3 perimeter descriptive report (Funkhouser AT, Fankhauser F: Temporal summation measurements with the Octopus 1-2-3 perimeter. Ger J Ophthalmol. 1994 Mar; 3(2):120-8) have been taken into consideration.

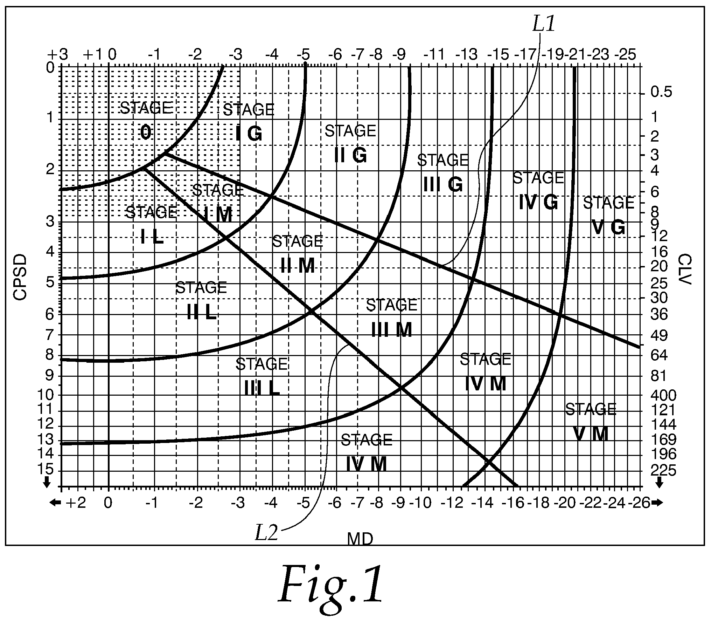

GSS uses the MD (mean deviation for Humphrey and mean defect for Octopus perimeters) and CPSD/PSD (standard deviation of the model corrected by Humphrey, which is practically identical to the square root of the Corrected Loss Variance for Octopus, CLV) on a chart, such as the one shown in Figure 1, of Cartesian coordinates.

The former is on the X axis and the latter is on the Y axis, with CPSD on the left and CLV on the right.

The distance between nearby values decreases progressively from left to right for MD and from the top to the bottom for CPSD in order to emphasize the area that should contain early glaucomatous defects (Figure 1).

The nomogram shows immediately the stage of the disease, which is defined by the intersection of the two values.

The chart is shown in Figure 1 and is divided arbitrarily into six different stages with curved lines:
- stage 0 (completely normal visual fields);
- stage 1 (very fine defects, such as mild depression of sensitivity in some points, etcetera);
- stage 2 (moderate field defects, such as a nasal passage or small scotomas, etcetera); stage
- stage 3 (evident defects, such as dense arched defects, etcetera);
- stage 4 (very advanced absolute defects)
- stage 5 (only islets of low remaining sensitivity).

"Enhanced Glaucoma Staging System (GSS2) for classifying Functional Damage in Glaucoma", P. Brusini and S. Filacorda, J Glaucoma, Vol. 15, Number 1, February 2006, describes a method known as "Enhanced Glaucoma Staging System" (GSS2) which uses mathematical formulas that define the curved lines that separate the stages in the Glaucoma Staging System described previously.

In GSS2 a new intermediate stage between stage 0 and stage 1 has been added, in order to include borderline cases.

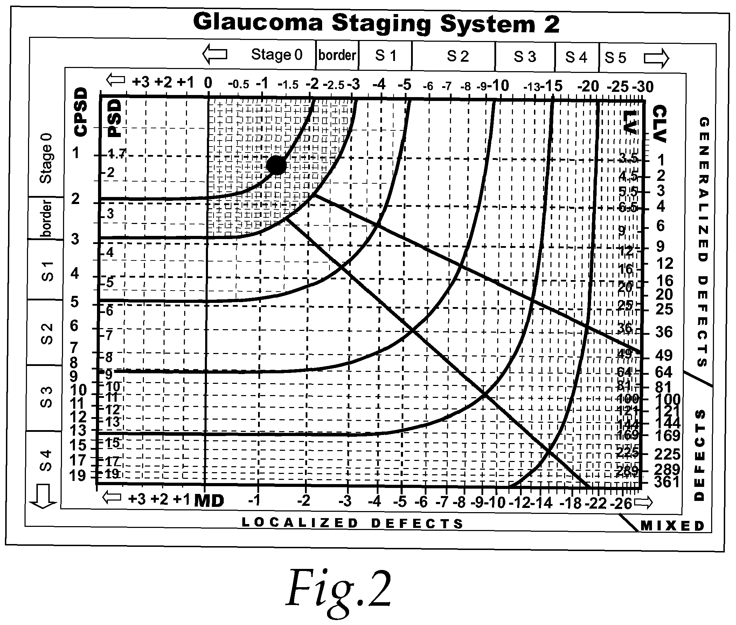

A chart for GSS2 is shown in Figure 2.

In recent years, a non-invasive diagnostic imaging technique has been developed which is known as "Optical Coherence Tomography" ("OCT"), and in particular so-called "Spectral-Domain OCT" ("SD- OCT") (Schuman JS, Hee MR, Puliafito CA et al: Quantification of Nerve Fiber Layer Thickness in Normal and Glaucomatous Eyes Using Optical Coherence Tomography. A Pilot Study. Arch Ophthalmol. 1995; 113(5):586-596) and has proved itself to be a valid aid in the diagnosis of glaucoma.

OCT allows to reveal retinal layers by observing laser light interference patterns, and in particular the fiber layer of the optic nerve ("Retinal Nerve Fiber Layer" or "RNFL"), which are relevant in glaucomatous pathologies since it has been found that in these circumstances the optic nerve fiber layer is subjected to a thinning.

WO 2016/071398 describes a method for classifying the defects of the nerve fiber layer deriving from glaucomatous damage.

The method comprises the following steps:
- receiving a pair of values comprising a first value of a first parameter relative to the measurement of the thickness of the layer of the fiber of the optic nerve of the superior quadrant of a subject, and a second value of a second parameter relative to the measurement of the thickness of the layer of the fiber of the optic nerve of the inferior quadrant of the subject,
- providing a definition of a reference structure comprising a plurality of sets of pairs of predetermined values, each one of said sets of pairs of predetermined values corresponding to a respective class of glaucomatous damage severity and location,
- identifying to which set of pairs of predetermined values of the reference structure said received pair of values belongs,
- determining the class of severity and location of the glaucomatous damage of the subject on the basis of the set of identified pairs of values to which said received pair of values belongs.

The measurements of the thickness of the nerve fibers forming the optic nerve of a patient can be predetermined with SD-OCT.

The reference structure can be a predetermined two-dimensional chart (for example, a two-dimensional Cartesian chart), in which values that can be assumed by one of the two parameters that represent the average RNFL of the thickness of the superior and respectively inferior quadrant are plotted on the axis of one of the two coordinates, for example the axis of the abscissas, and values that can be assumed by the other one of said two parameters that can be obtained from the SD-OCTs are plotted on the other axis, the axis of the ordinates.

These two parameters are directly affected by glaucomatous damage.

One chart that is used is preferably a chart that is predetermined in a specific manner for the age and/or race of the patient.

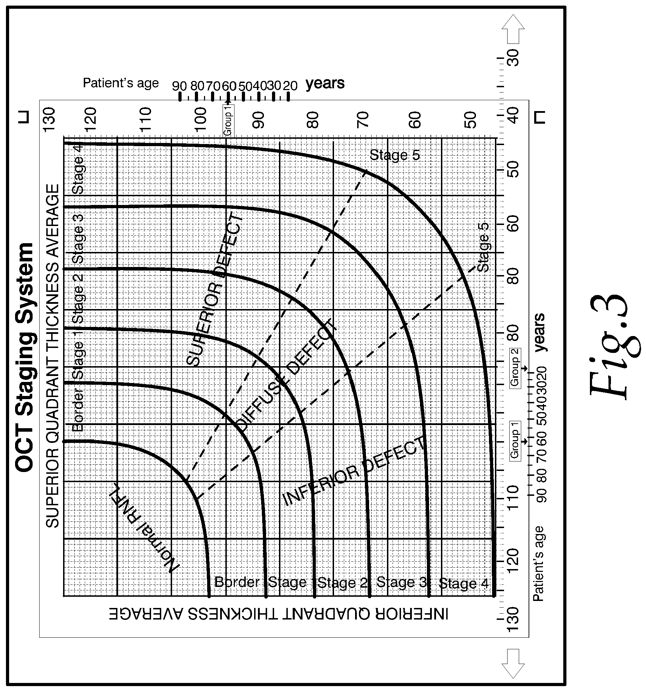

Such a chart is shown in Figure 3.

The chart that is used comprises advantageously a grid of lines that divides the portion of plane defined by the two Cartesian axes into a plurality of adjacent areas, which define different stages of different types of glaucomatous damage, i.e., different classes of severity of the glaucomatous damage, as shown in Figure 3.

The grid lines can be straight line segments, circular arcs, curved lines, as a function of the type of representation used for the values of the two parameters on the two Cartesian axes.

In particular, the line grid defines a sector around the left superior apex of the chart (which corresponds to values of the "RNFL average of the thickness of the superior quadrant" and "RNFL average of the thickness of the inferior quadrant" parameters that corresponds to a normal condition of the patient), five bands which, moving away from the normality sector toward the inferior right apex of the chart, correspond in this order to a limiting or border condition and to four successive stages of glaucomatous damage, and a sector around the right inferior apex of the chart that corresponds to a fifth stage of damage amount.

Consequently, the line grid defines, besides the normality situation, six stages of progressive severity of the glaucomatous damage due to the damage to the optic nerve.

In other words, the chart, provided with a grid of lines and straight line segments, represents a reference structure comprising a plurality of contiguous areas in the plane, each comprising a corresponding plurality of sets of pairs of predetermined values of the "RNFL average of the thickness of the superior quadrant" and "RNFL average of the thickness of the inferior quadrant" parameters, where each area corresponds to a respective class of severity and location of the glaucomatous damage due to damage to the optic nerve.

Considering to plot on a Cartesian plane the values of the "RNFL average of the thickness of the superior quadrant" parameter on one axis, for example the axis of the abscissas, and the values of the "RNFL average of the thickness of the inferior quadrant" parameter on the other axis, for example the axis of the ordinates, the shape and/or position (in said Cartesian plane) of the grid of lines that define the different stages of different types of glaucomatous damage, the shape and/or the position (in said Cartesian plane) of the straight line segments that define the locations of the damage, and/or the trend of the values of the "RNFL average of the thickness of the superior quadrant" and "RNFL average of the thickness of the inferior quadrant" parameters with respect to the grid of lines and to the straight line segments depend on the age and race of the patient. Adapted software takes into account the age of the patient and the type of OCT used for the test.

Bearing in mind that, in glaucoma, the damage to the visual field and the damage to the optic nerve do not always go hand in hand (damage to the nerve fibers often precedes damage to the visual field, although sometimes the opposite occurs), the need is felt to define a standard method for classifying and analyzing the degree of the global glaucomatous damage.

The aim of the present invention is to provide a method and a system which, by integrating the information originating from different tests, allow to evaluate the extent of the glaucomatous damage as a whole, and which besides classifying the global damage in rising severity stages also indicate whether the damage affects predominantly the visual field, the nerve fibers ("preperimetric glaucoma") or if the two types of damage are more or less equivalent.

Within this aim, an object of the present invention is to provide a method that assists in choosing a treatment of the disease that is more aggressive as the severity of the glaucomatous damage increases.

Another object of the present invention is to provide a method that simplifies considerably the interpretation of the two tests (visual field and OCT) and is undeniably useful especially for less expert eye specialists, allowing them to obtain in few seconds reliable information on the presence and severity of the damage in glaucomatous patients.

Another object of the present invention is to provide a method that allows to quantify in a standardized and objective manner the glaucomatous damage that is present at a certain moment.

Another object of the present invention is to provide a method that allows to file in the medical record of the patient (both paper-based and computerized) in an extremely concise and simplified manner data related to the functional and structural glaucomatous damage that is present.

Another object of the present invention is to provide a method that can be used to standardize the criteria for inclusion of patients affected by glaucoma in scientific studies that require the recruitment of patients with homogeneous damage.

Another object of the present invention is to provide a method that allows to clearly display on a chart any progression of glaucomatous disease.

Another object of the present invention is to provide a method that can be used to explain graphically to the patient affected by glaucoma his clinical situation.

Another object of the present invention is to provide a method that can be useful for medical-legal purposes in relation to glaucomatous damage.

This aim and these and other objects that will become better apparent hereinafter are achieved by a method for classifying damage due to glaucoma of a patient, on the basis of an existing data set which comprises:
- a value MD of average loss of sensitivity of a visual field of said patient with respect to a subject without glaucoma of similar age,
- a value PSD of standard deviation of the MD of said patient,
- a value RNFL of average thickness of the layer of fibers of the optic nerve in the inferior quadrant of said patient,
- a value RNFL of average thickness of the layer of fibers of the optic nerve in the superior quadrant of said patient,
   said method comprising the following steps:
   1) determining a score that indicates the severity of the damage to the visual field of said patient on the basis of said MD and PSD values, in stages of rising severity of the damage to the visual field,
   2) determining a score that indicates the severity of the damage to the optic nerve of said patient on the basis of said values RNFL of average thickness of the layer of fibers of the optic nerve in the inferior and superior quadrants of said patient,
   3) determining a score of the global damage due to glaucoma of said patient on the basis of said score that indicates the severity of the damage to the visual field and of said score indicating the severity of the damage to the optic nerve of said patient.

The method according to the present invention is termed "Global Glaucoma Staging System" (GGSS).

Step 1) of the method of the present invention can be performed by using a predetermined Cartesian coordinate chart with X and Y axes, with MD on the X axis and PSD on the Y axis.

Said chart comprises a partitioning grid which defines contiguous classes of severity of the damage to the visual field with scores from 1 to more than 5, identifying on said coordinates said MD and PSD values of the patient, and detecting the position on the chart of a point that is uniquely defined by said MD and PSD values of the patient, said uniquely defined point being uniquely associated with a score in the range from 0 to more than 5 that indicates the severity of the damage to the visual field of said patient.

Step 1) of the method of the present invention can be performed by using a method described in Italian Patent Application No. UD95A000007, in Clinical use of a new method for visual field damage classification in glaucoma, P. Brusini, European Journal of Ophthalmology, Vol. 6, 1996, p. 402-407, or in Enhanced Glaucoma Staging System (GSS2) for classifying Functional Damage in Glaucoma, P. Brusini and S. Filacorda, J Glaucoma, Vol. 15, Number 1, February 2006 cited above.

The data of step 1) can be obtained using adapted software in which the MD and PSD values are entered, allowing to obtain the staging of the severity of the damage in continuous form instead of in six stages as present in the paper chart.

The charts that can be used in step 1) are, for example, of the type shown in Figures 1 and 2.

Step 2) of the method of the present invention can be performed using a predetermined chart with X and Y Cartesian coordinates, with the thickness of the fibers of the optic nerve in the superior quadrant on the X axis and the thickness of the fibers of the optic nerve in the inferior quadrant on the Y axis.

Said chart comprises a partitioning grid which defines contiguous classes of severity of the damage to the optic nerve with scores from 1 to more than 5, identifying on said coordinates said values of optic nerve fiber thickness in the superior and inferior quadrants of the patient, and detecting the position on the chart of a point that is uniquely defined by said values of optic nerve fiber thickness in the superior and inferior quadrants of the patient, said uniquely defined point being uniquely associated with a score in the range from 0 to more than 5 which indicates the severity of the damage to the optic nerve of said patient.

Step 2) of the method of the present invention can be performed using the method described in WO 2016/071398.

The chart used in step 2) can be obtained using adapted software in which the data of the patient, the type of OCT used and the thickness of the peripapillary nerve fibers in the superior and inferior quadrants are entered. In this manner the loss of nerve fibers caused by aging and the differences existing among the various OCTs in use are also taken into account.

A chart that can be used in step 2) is, for example, of the type shown in Figure 3.

Step 3) of the method of the present invention can be performed by using a predetermined Cartesian coordinate chart with X and Y axes, with a score that indicates the severity of the damage to the visual field on the X axis and a score that indicates the severity of the damage to the optic nerve on the Y axis.

Such chart comprises a partitioning grid which defines contiguous classes of severity of the global damage caused by glaucoma with scores from 0 to more than 5, which indicate the severity of the overall damage in stages of severity that increase from normality stage 0 to terminal damage stage 5, identifying on said coordinates said scores that indicate the severity of the damage to the visual field and the severity of the damage to the optic nerve of said patient determined in steps 1) and 2), and detecting the position on the chart of a point that is defined uniquely by said scores that indicate the severity of the damage to the visual field and the severity of the damage to the optic nerve of said patient, said uniquely defined point being uniquely associated with a score in the range from 0 to more than 5 which indicates the severity of the global damage caused by the glaucoma of said patient.

The chart used in step 3) is obtained by entering the values that correspond to the stage of damage to the visual field on the abscissas (continuous value from 0 to more than 5) and the values related to the damage to the nerve fibers (continuous values from 0 to more than 5) on the ordinates in adapted software. The intersection of the two values indicates the stage of the global damage. New software allows to calculate the global damage directly by entering only the data necessary for the staging of the damage to the visual field (MD and PSD) and the damage to the nerve fibers (thickness of the nerve fibers in the superior and inferior quadrants).

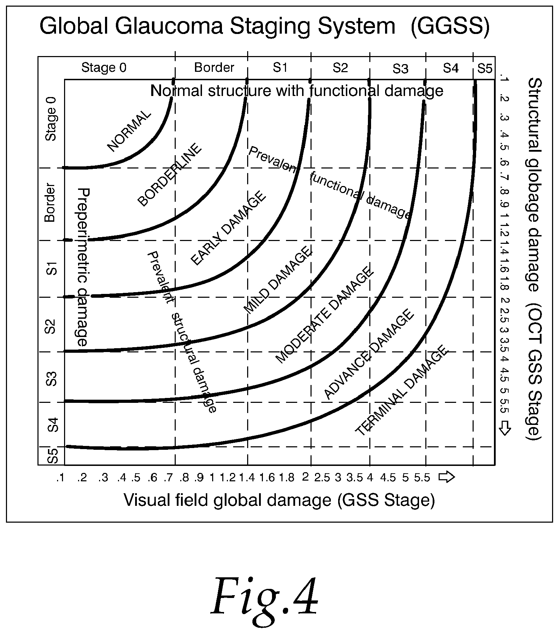

A chart that can be used in step 2) is for example of the type shown in Figure 4.

As is evident from the present description, the method according to the present invention is not a method for surgical or therapeutic treatment or a diagnostic method applied to the human body, but it merely uses already existing data related to glaucoma damage of a subject in order to classify the damage.

The Global Glaucoma Staging System (GGSS) of the present invention is a method for classifying the damage due to the glaucoma, which takes into consideration simultaneously the damage to the visual field and the damage affecting the fibers of the optic nerve.

In order to obtain the staging of the global damage with the method of the present invention it is possible to use data deriving from two previous classification methods, the first of which gives indications on the severity of the damage to the visual field while the second one gives indications on the severity of the damage to the optic nerve.

The first method can be the Glaucoma Staging System (GSS), preferably the Glaucoma Staging System 2 (GSS2) mentioned above, which uses a Cartesian chart that enters in the abscissas the Mean Deviation (MD = average loss of sensitivity of a visual field, with respect to a subject of the same age) and in the ordinates the Pattern Standard Deviation (PSD = standard deviation of MD).

The intersection of these two indicates the severity of the damage to the visual field (divided into 6 stages plus normality stage 0) and the type of damage present (generalized, localized or mixed).

This method is described in UD95A000007, in Clinical use of a new method for visual field damage classification in glaucoma, P. Brusini, European Journal of Ophthalmology, Vol. 6, 1996, p. 402-407, or in Enhanced Glaucoma Staging System (GSS2) for classifying Functional Damage in Glaucoma, P. Brusini and S. Filacorda, J Glaucoma, Vol. 15, Number 1, February 2006 mentioned above.

The second method can be the OCT Glaucoma Staging System mentioned above, which also uses a Cartesian chart taking into consideration the thickness of the fibers of the optic nerve (measured with an Optical Coherent Tomograph, OCT) in the superior quadrant (in the abscissas) and in the inferior quadrant (in the ordinates). The intersection of these two values provides indications on the severity of the damage to the optic nerve (6 stages plus stage 0) and on the location of said damage (superior, inferior or widespread).

This method is described in WO 2016/071398.

The GGSS method for classifying the damage due to glaucoma of the present invention uses a Cartesian chart divided in 6 stages plus normality stage 0, in which data related to the severity of the damage to the visual field, which are determined with the first method, are entered in the abscissas no longer as a stage (which has fixed limits), but as continuous variable or score (from 0 to more than 5), and the values that can be referred to the damage to the nerve fibers, which are determined with the second method, are entered in the ordinates, and are applied, in this case also, as continuous variable or score from 0 to more than 5.

The intersection of these scores indicates the severity of the global damage due to glaucoma. Therefore, neither the type of damage to the visual field nor the location of the damage to the nerve fibers are taken into consideration.

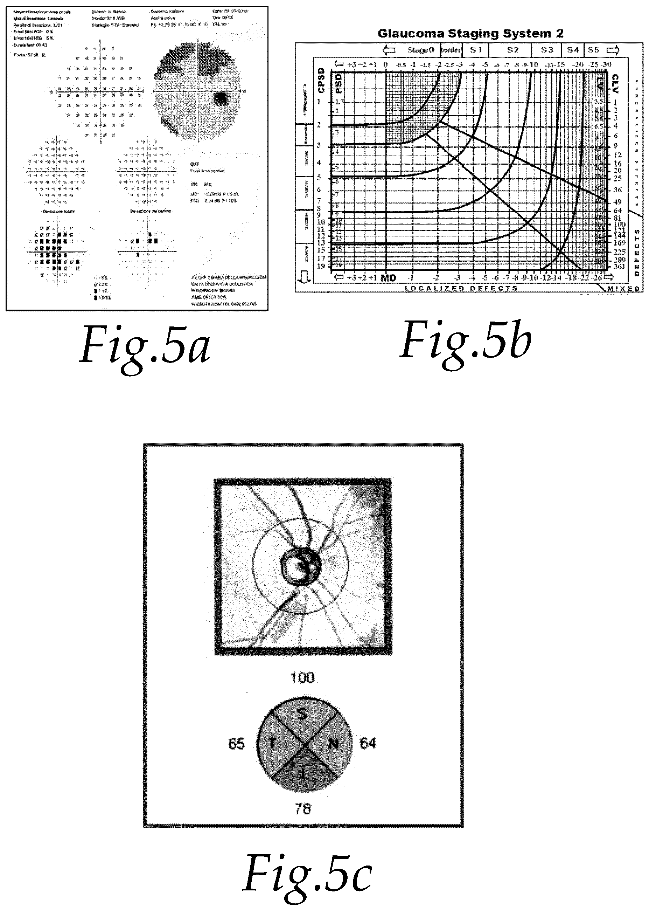

Figures 5a to 5e show a first example, related to a first clinical case, by way of non-limiting example of the present invention.

Figure 5a shows graphically the outcome of the test of the visual field (CAP) of the patient in question.

Figure 5b shows the patient's GSS2 derived from the CAP of Figure 5a. In the case considered in the example, GSS2=2.3.

Figure 5c shows graphically the outcome of the OCT (RNFL) test, with the superior quadrant equal to 100 and the inferior quadrant equal to 78. The upper photograph of Figure 5c shows the map of the RNFL deviation.

Figure 5d shows its OCT GSS deriving from the OCT test of Figure 5c. In the case being considered, OCT GSS=2.8.

Figure 5e shows the GGSS graphically. Specifically, GGSS is equal to stage 2, i.e., "mild damage", with equivalent functional and structural damage.

Figures 6a to 6e show a second example, related to a second clinical case, by way of non-limiting example of the present invention.

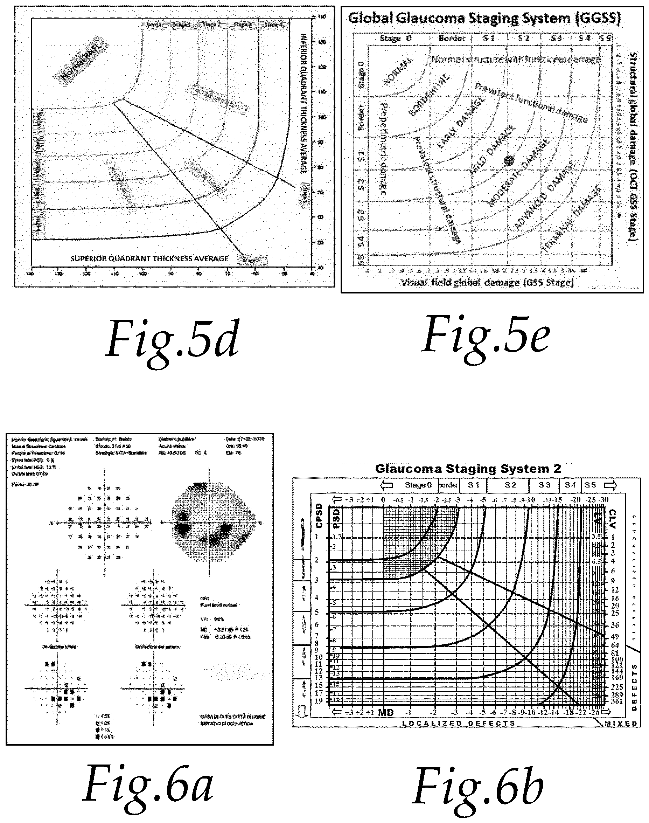

Figure 6a shows graphically the outcome of the visual field test (CAP) of the patient in question.

Figure 6b shows its GSS2 deriving from the CAP of Figure 6a. In the example case, GSS2=2.7.

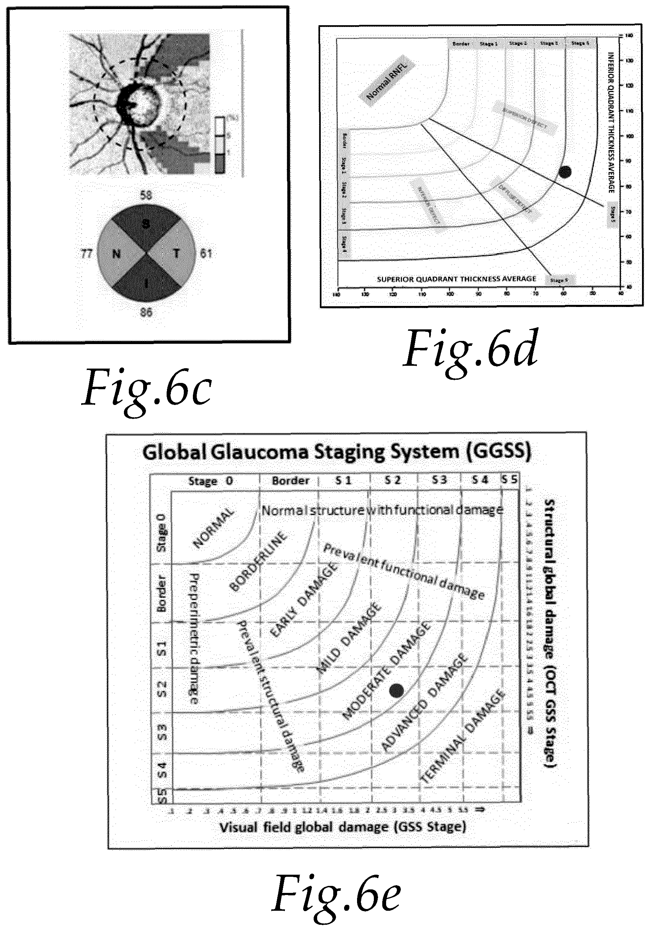

Figure 6c shows graphically the outcome of the OCT (RNFL) test, with the superior quadrant equal to 58 and the inferior quadrant equal to 86. The upper photograph of Figure 6c shows the map of the RNFL deviation.

Figure 6d shows its OCT GSS deriving from the OCT test of Figure 6c. In the case being considered, OCT GSS=4.3.

Figure 6e shows the GGSS graphically. Specifically, GGSS is equal to stage 3, i.e., "moderate damage", with equivalent functional and structural damage.

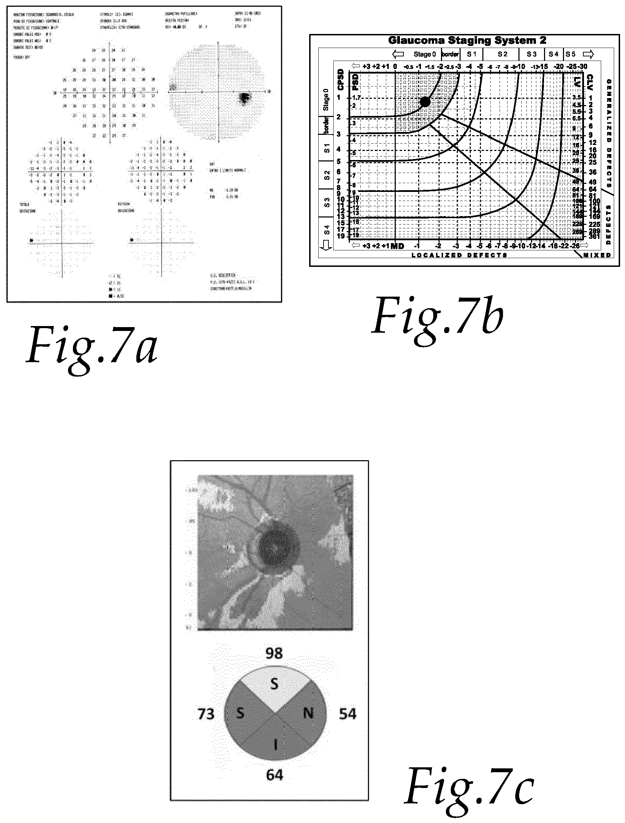

Figures 7a to 7e show a third example, related to a third clinical case, by way of non-limiting example of the present invention.

Figure 7a shows graphically the outcome of the visual field test (CAP) of the patient in question.

Figure 7b shows its GSS2 deriving from the CAP of Figure 7a. In the example case, GSS2=0.6.

Figure 7c shows graphically the outcome of the OCT (RNFL) test, with the superior quadrant equal to 98 and the inferior quadrant equal to 64. The upper photograph of Figure 7c shows the map of the RNFL deviation.

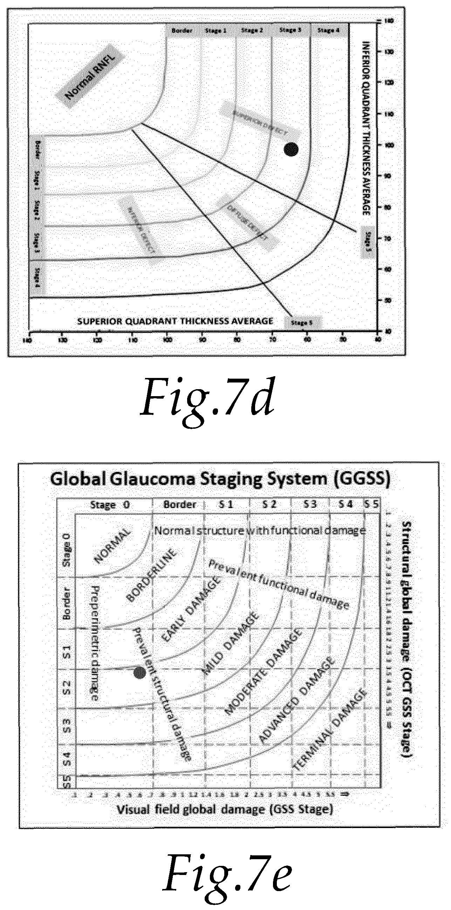

Figure 7d shows its OCT GSS deriving from the OCT test of Figure 7c. In the case being considered, OCT GSS=3.6.

Figure 7e shows the GGSS graphically. Specifically, GGSS is equal to stage 2, i.e., "mild damage", with preperimetric damage.

All data (values and scores of patients) necessary for using the method that is the subject matter of the present patent application and all the data obtained from the use of said method are collected and saved on a local and/or remote cloud server.

The disclosures in Italian Patent Application No. 102019000006120, from which this application claims priority, are incorporated by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A method for classifying the damage due to the glaucoma of a patient on the basis of an existing data set which comprises:
- a value MD of average loss of sensitivity of a visual field of said patient with respect to a subject without glaucoma of similar age,
- a value PSD of standard deviation of the MD of said patient,
- a value RNFL of average thickness of the layer of fibers of the optic nerve in the inferior quadrant of said patient, and
- a value RNFL of average thickness of the layer of fibers of the optic nerve in the superior quadrant of said patient,
said method comprising the following steps:
1) determining a score that indicates the severity of the damage to the visual field of said patient on the basis of said MD and PSD values, in stages of rising severity of the damage to the visual field,
2) determining a score that indicates the severity of the damage to the optic nerve of said patient on the basis of said values RNFL of average thickness of the layer of fibers of the optic nerve in the inferior and superior quadrants of said patient,
3) determining a score of the global damage due to glaucoma of said patient on the basis of said score that indicates the severity of the damage to the visual field and of said score indicating the severity of the damage to the optic nerve of said patient, which have been determined in steps 1) and 2).

2. The method according to claim 1, wherein step 1) is performed by using a predetermined Cartesian coordinate chart with X and Y axes, with MD on the X axis and PSD on the Y axis, said chart comprising a partitioning grid which defines contiguous classes of severity of the damage to the visual field with scores from 1 to over 5, identifying on said coordinates said MD and PSD values of the patient, and detecting the position on the chart of a point that is uniquely defined by said MD and PSD values of the patient, said uniquely defined point being uniquely associated with a score in the range from 0 to over 5 that indicates the severity of the damage to the visual field of said patient.

3. The method according to claim 1 or 2, wherein step 2) is performed by using a predetermined chart with X and Y Cartesian coordinates, with the thickness of the fibers of the optic nerve in the superior quadrant on the X axis and the thickness of the fibers of the optic nerve in the inferior quadrant on the Y axis, said chart comprising a partitioning grid which defines contiguous classes of severity of the damage to the optic nerve with scores from 1 to over 5, identifying on said coordinates said values of optic nerve fiber thickness in the superior and inferior quadrants of the patient, and by detecting the position on the chart of a point that is uniquely defined by said values of optic nerve fiber thickness in the superior and inferior quadrants of the patient, said uniquely defined point being uniquely associated with a score in the range from 0 to over 5 which indicates the severity of the damage to the optic nerve of said patient.

4. The method according to claim 1, wherein step 3) is performed by using a predetermined Cartesian coordinate chart with X and Y axes, with a score that indicates the severity of the damage to the visual field on the X axis and a score that indicates the severity of the damage to the nerve fibers of the optic nerve on the Y axis, said chart comprising a partitioning grid which defines contiguous classes of severity of the global damage caused by glaucoma with scores from 0 to over 5 which indicate the severity of the overall damage in stages of severity that increase from normality stage 0 to terminal damage stage 5, identifying on said coordinates said scores that indicate the severity of the damage to the visual field and the severity of the damage to the optic nerve of said patient, and detecting the position on the chart of a point that is uniquely defined by said scores that indicate the severity of the damage to the visual field and the severity of the damage to the optic nerve of said patient, said uniquely defined point being uniquely associated with a score in the range from 0 to more than 5 which indicates the severity of the global damage caused by the glaucoma of said patient.

5. The method according to one or more of the preceding claims, **characterized in that** at least one of said steps 1), 2) and 3) is performed by means of software.

6. The method according to one or more of the preceding claims, **characterized in that** each value and each score of said patient is saved on a local and/or remote cloud server.
